(19) Europäisches Patentamt
European Patent Office
Office européen des brevets

(11) Veröffentlichungsnummer: **0 254 180 B1**

# (12) EUROPÄISCHE PATENTSCHRIFT

(45) Veröffentlichungstag der Patentschrift: **16.10.91**

(51) Int. Cl.[5]: **C07C 45/50**, C07C 47/02

(21) Anmeldenummer: **87110130.9**

(22) Anmeldetag: **14.07.87**

## (54) Verfahren zur kontinuierlichen Hydroformylierung olefinisch ungesättigter Verbindungen.

(30) Priorität: **25.07.86 DE 3625261**

(43) Veröffentlichungstag der Anmeldung:
**27.01.88 Patentblatt 88/04**

(45) Bekanntmachung des Hinweises auf die Patenterteilung:
**16.10.91 Patentblatt 91/42**

(84) Benannte Vertragsstaaten:
**AT BE DE ES FR GB IT NL SE**

(56) Entgegenhaltungen:
**EP-A- 0 158 196**
**US-A- 3 271 458**

(73) Patentinhaber: **BASF Aktiengesellschaft**
**Carl-Bosch-Strasse 38**
**W-6700 Ludwigshafen(DE)**

(72) Erfinder: **Zehner, Peter, Dr.**
**Erich-Kaestner-Strasse 15**
**W-6700 Ludwigshafen(DE)**
Erfinder: **Hoffmann, Herwig, Dr.**
**Knietschstrasse 21**
**W-6710 Frankenthal(DE)**
Erfinder: **Richter, Wolfgang, Dr.**
**Am Huettenwingert 16**
**W-6706 Wachenheim(DE)**
Erfinder: **Stuetzer, Dieter, Dr.**
**Carl-Zimmermann-Strasse 21**
**W-6724 Dudenhofen(DE)**
Erfinder: **Strohmeyer, Max, Dr.**
**Woogstrasse 41**
**W-6703 Limburgerhof(DE)**
Erfinder: **Walz, Helmut, Dr.**
**Osloer Weg 18**
**W-6700 Ludwigshafen(DE)**
Erfinder: **Weippert, Erich, Dr.**
**Koenigshofer Strasse 30**
**W-6800 Mannheim 51(DE)**

**EP 0 254 180 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur kontinuierlichen Hydroformylierung von olefinisch ungesättigten Verbindungen, unter Einleitung des Olefins im unteren Bereich des Reaktors, bei 1 bis 40 bar und 50 bis 140° C mit Hilfe von Rhodium-Komplexverbindungen als Katalysatoren in einem Hydroformylierungsreaktor mit einer flüssigen Reaktionszone, die etwa 60 bis 85 % des Reaktorvolumens beträgt, Entfernung der gasförmigen Verfahrensprodukte und Reaktionspartner aus dem Hydroformylierungsreaktor, Abtrennung der Verfahrensprodukte und größtenteils Rückführung des verbleibenden Gases in den Reaktor nach der Kreisgasfahrweise.

Die Hydroformylierungsreaktion olefinisch ungesättigter Verbindungen mittels Rhodium-Katalysatoren ist allgemein bekannt, z.B. aus Chemical Engineering, Dezember 1977, S. 110 bis 115 oder den deutschen Offenlegungsschriften 33 01 591, 17 93 069 und 11 86 455. Weiterhin ist z.B. aus den DE-A-27 15 685 und 31 14 147 bekannt, die sogenannte Kreisgasfahrweise anzuwenden, nach der man die Verfahrensprodukte, d.h. vorwiegend Aldehyde und daneben die entsprechenden Alkohole, zusammen mit gasförmigen Reaktionspartnern dampfförmig aus dem Reaktor austrägt, die Produkte aus dem Gasstrom abtrennt und die verbleibenden Gase, die im wesentlichen Kohlenmonoxid, Wasserstoff und nicht umgesetztes Olefin enthalten, größtenteils wieder in die Flüssigkeitszone des Reaktors zurückführt.

Obwohl dieses Verfahren gute Ergebnisse liefert, lag der Erfindung die Aufgabe zugrunde, die Ausbeute an den Verfahrensprodukten, also den Aldehyden, weiter zu verbessern.

Demgemäß wurde ein Verfahren zur kontinuierlichen Hydroformylierung von olefinisch ungesättigten Verbindungen, unter Einleitung des Olefins im unteren Bereich des Reaktors, bei 1 bis 40 bar und 50 bis 140° C mit Hilfe von Rhodium-Komplexverbindungen als Katalysatoren in einem Hydroformylierungsreaktor mit einer flüssigen Reaktionszone, die etwa 60 bis 85 % des Reaktorvolumens beträgt, Entfernung der gasförmigen Verfahrensprodukte und Reaktionspartner aus dem Hydroformylierungsreaktor, Abtrennung der Verfahrensprodukte und größtenteils Rückführung des verbleibenden Gases in den Reaktor nach der Kreisgasfahrweise gefunden, das dadurch gekennzeichnet ist, daß man 20 bis 80 Vol.% des Kreisgases dem Hydroformylierungsreaktor oberhalb der flüssigen Reaktionszone und/oder unterhalb der Flüssigkeitsoberfläche im oberen Viertel der flüssigen Reaktionszone zuführt.

Nach dem erfindungsgemäßen Verfahren wird das Kreisgas nicht wie bislang üblich am Boden des Reaktors in die Flüssigkeitszone, die etwa 2/3 des Reaktionsvolumens ausmacht, zugeführt, sondern 20 bis 80 Vol.% des Kreisgases direkt in den Dampfraum, oberhalb des aus flüssigen und gasförmigen Bestandteilen bestehenden Hydroformylierungsgemisches und/oder unterhalb der Flüssigkeitsoberfläche im oberen Viertel der flüssigen Reaktionszone, wobei die Zugabe unterhalb der Flüssigkeitsoberfläche besonders bevorzugt wird. Gute Ausbeutesteigerungen können insbesondere erzielt werden, wenn man 40 bis 60 Vol.% des Kreisgases in der erfindungsgemäßen Weise zuführt. Das restliche Kreisgas kann entweder am Boden des Reaktors zugegeben werden oder vorteilhaft in Teilmengen in Ebenen unterschiedlicher Höhe. Daß die erfindungsgemäße Maßnahme eine Ausbeuteerhöhung bedingt, ist überraschend, weil zu erwarten war, daß eine Rückführung des gesamten Kreisgases zusammen mit dem Frischgas am Boden des Reaktors die Aldehydbildung wegen des größeren Angebotes an Ausgangsstoffen begünstigt.

Der Effekt der erfindungsgemäßen Verfahrensweise tritt besonders deutlich zutage, wenn Umsetzungen im großtechnischen Maßstab durchgeführt werden. Im allgemeinen gilt die Regel, daß der Effekt umso größer ist, je größer die Dimension des Reaktors ist. Daher sind Reaktortypen mit einem Durchmesser ab 1 m und einer Höhe von mindestens 5 m besonders bevorzugt. Zweckmäßigerweise liegen die Dimensionen im Bereich von 1 bis 5 m, insbesondere 2 bis 4 m Durchmesser und 5 bis 30 m, insbesondere 15 bis 30 m Höhe.

Die erfindungsgemäße Arbeitsweise ermöglicht, daß durch den Einsatz höherer Rhodiummengen weitere Umsatzsteigerungen erzielt werden können. Während bislang durch Erhöhung der Rhodiumkonzentration, die üblicherweise 100 bis 150 ppm, bezogen auf das flüssige Reaktionsgemisch, beträgt, keine signifikanten Umsatzsteigerungen möglich waren, wurde nun gefunden, daß sich eine höhere Rhodiummenge auf den Umsatz auswirkt, wobei der Umsatz proportional zur Rhodiummenge ansteigt. Vorzugsweise werden daher Rhodiumkonzentrationen im Bereich von ca. 120 bis 500, insbesondere 150 bis 500 ppm, bezogen auf das flüssige Reaktionsgemisch, gewählt.

Gemäß einer weiteren Ausführungsform des Verfahrens kann man die für die Hydroformylierung benötigte $CO/H_2$-Gasmenge aufteilen und die Teilmengen in Ebenen unterschiedlicher Höhe der flüssigen Reaktionszone zuführen. Beispielsweise kann man ca. 20 bis 80, insbesondere 20 bis 40 Vol.% des $CO/H_2$-Gases am Boden des Reaktors zuführen und den Rest auf mindestens einer Ebene, vorteilhaft 3 bis 5 Ebenen unterschiedlicher Höhe oberhalb des unteren Fünftels der flüssigen Reaktionszone. So ist es z.B. möglich, ca. 20 bis 40 Vol.% des $CO/H_2$-Gemisches am Boden des Reaktors einzuleiten und den Rest in

das zweite, dritte und vierte Viertel der flüssigen Reaktionszone.

Bei Zuführung des gesamten Gases am Boden des Reaktors gemäß dem Stand der Technik bildet sich bis zur Oberfläche des Hydroformylierungsgemisches ein CO-Gradient von ca. 10 % aus, der durch die beschriebene Zudosierung auf etwa 2 % abgesenkt werden kann.

Durch die erfindungsgemäße Zufuhr des Kreisgases in den Reaktor sowie ggf. Aufteilung der $CO/H_2$-Gemisches soll vermieden werden, daß größere Gasansammlungen an Kreisgas oder Frischgas auftreten. Es erübrigt sich daher, weitere Zugabemöglichleiten der Gasteilmengen aufzuführen, da es der Fachmann aufgrund dieser Lehre in der Hand hat, die Gasmenge sinnvoll aufzuteilen.

Abgesehen von der erfindungsgemäßen Verbesserung kann man das Verfahren sowohl hinsichtlich der Hydroformylierungsstufe als auch der Abtrennung der Verfahrensprodukte aus dem Gasstrom in an sich bekannter Weise durchführen, so daß detaillierte Ausführungen hierzu entbehrlich sind. Im folgenden seien daher nur einige grundsätzliche Erläuterungen gegeben.

Die Hydroformylierung der Olefine wird bei einem Druck von 1 bis 40 bar und einer Temperatur von 50 bis 140 °C vorgenommen. Als Olefine kommen im allgemeinen α-Olefine in Betracht, die unter den Reaktionsbedingungen inerte funktionelle Gruppen enthalten können, beispielsweise Allylalkohol, Allylacetat, niedermolekulare Acrylsäureester und Acroleinacetale.

Vorzugsweise können niedere Olefine mit 2 bis 4 C-Atomen, z.B. Ethylen, Propylen und But-1-en umgesetzt werden, da die gebildeten Aldehyde einen hohen Partialdruck besitzen, so daß sich ein dampfförmiger Austrag lohnt.

Als Katalysatoren für die Hydroformylierung kommen Rhodium-Komplexverbindungen in Betracht, die schwer flüchtige Verbindungen I

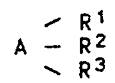

I

in der A für Phosphor, Arsen, Antimon oder Wismut steht und in der $R^1$-$R^3$ organische Reste bedeuten, als Liganden enthalten.

In der Regel werden die Verbindungen I in 3- bis 500fachem molarem Überschuß über das Rhodium eingesetzt und der Rhodium-Komplex in situ im Hydroformylierungsgemisch aus Rhodiumsalzen, z.B. dem Acetat gebildet. Natürlich kann auch der separat hergestellte, fertige Rhodium-Komplex zugesetzt werden.

Die Wahl der Liganden I kann sich im Einzelfall nach den speziellen Zielsetzungen richten. Im allgemeinen werden Triorganophosphorverbindungen wie Triaryl- oder Aryl-alkylphosphine sowie Trialkyl-, Triaryl- oder Aryl-alkylphosphite verwendet. Trialkylphosphine sind im allgemeinen weniger gut geeignet. Besonders bevorzugte Organophosphorverbindungen sind Triarylphosphine wie Triphenyl- oder Tritolylphosphin oder Aryl-alkylphosphine wie Diphenyl-$C_1$-$C_8$-Alkylphosphine.

Das $CO/H_2$-Molverhältnis kann je nach der Hydroformylierungsaufgabe zwischen etwa 10:90 bis 90:10 liegen. Vorteilhaft liegt es zwischen 45:55 und 55:45, wenn Aldehyde als Verfahrensprodukte erwünscht sind, was im allgemeinen der Fall ist.

Die Abtrennung der Verfahrensprodukte aus dem gasförmigen Austrag kann in bekannter Weise, z.B. wie in Chemical Engineering, 1977, S. 110ff oder der DE-OS 31 14 147 beschrieben, erfolgen. So kann man das Gasgemisch in einem Kühler soweit abkühlen, daß sich die Produkte weitgehend verflüssigen und mittels eines Abscheiders abgetrennt werden können. Diese Flüssigphase kann anschließend vor der destillativen Aufarbeitung zwecks Zurückgewinnung darin gelöster Olefine durch eine Entgasungskolonne geleitet werden. Die aus dem Abscheider sowie der Entgasungskolonne erhaltenen Gase, die im wesentlichen nicht umgesetztes Olefin, das entsprechende Paraffin sowie CO und $H_2$ enthalten, werden größtenteils komprimiert und als Kreisgas in den Reaktor zurückgeführt. Damit die Menge der nicht verwertbaren Gase wie Paraffin und Stickstoff nicht ständig zunimmt, muß ein Teil des Kreisgases als Abgas aus dem System entfernt werden. Vorteilhaft kann man für niedere Olefine, z.B. Ethylen, die in der DE-OS 31 14 147 beschriebene Verfahrensweise wählen, wonach der gasförmige Reaktionsaustrag ohne zu kühlen und zu entspannen einer Destillationskolonne zugeführt wird, die Kopffraktion dieser Kolonne in einem Kühler soweit gekühlt wird, daß die überwiegenden Teile der darin enthaltenen Aldehyde kondensiert und das Kondensat in einem Abscheider in Gas- und Flüssigphase getrennt wird. Die Flüssigphase wird zurück in die Destillationskolonne geleitet und der Aldehyd als flüssiger Sumpfabzug und/oder dampfförmiger Seitenabzug entnommen. Die Gasphase wird, wie zuvor beschrieben, nach Abtrennung von Abgas, das etwa 1 bis 5 Vol.% des Kreisgases ausmacht, und Ausgleich des Druckverlustes mittels eines Kompressors in den Hydroformylierungsreaktor zurückgeführt und zwar in der Weise, daß mindestens 20 Vol.% oberhalb

der Flüssigkeitsoberfläche oder im oberen Viertel der flüssigen Reaktionszone des Reaktors eintreten.

Das erfindungsgemäße Verfahren gestattet gegenüber der herkömmlichen Fahrweise eine Erhöhung der Aldehydausbeute von mindestens etwa 5 bis 10 %, wobei das n/iso-Verhältnis nach den bisherigen Beobachtungen unverändert bleibt.

Beispiel 1

Hydroformylierung von Propylen

1a) In einem Versuchsreaktor, der zu ca. 2/3 mit Butyraldehyd-Kondensationsprodukten gefüllt war, wurden stündlich 488 kg Propylen, 228,4 $Nm^3$ CO und 270 $Nm^3$ $H_2$ gegeben und wie üblich in Gegenwart von 153 ppm Rhodium und 3,8 Gew.% Triphenylphosphin, bezogen auf das Reaktionsgemisch (Verhältnis Rhodium/Triphenylphosphin = rund 98:1) bei 110 °C und 16 bar hydroformyliert. Die Verfahrensprodukte wurden dem Reaktor zusammen mit nicht umgesetzten Einsatzstoffen gasförmig entnommen und in bekannter Weise abgetrennt. 40 Vol.% des Kreisgases wurden dem Reaktor ca. 15 % unterhalb der Oberfläche der flüssigen Reaktionszone wieder zugeführt.

1b) Dieser Versuch wurde unter den gleichen Bedingungen, jedoch Zuführung des Kreisgases am Boden des Reaktors in das flüssige Hydroformylierungsgemisch wiederholt, wobei weniger CO und $H_2$ verbraucht wurden.

Die Ergebnisse beider Versuche sind in der folgenden Tabelle zusammengestellt.

| | Versuch 1a erfindungsgemäß | Versuch 1b Vergleich |
|---|---|---|
| Propylen - Umsatz, % | 92 | 85,7 |
| davon zu | | |
| n-Butyraldehyd, % | 75,3 | 69,9 |
| iso-Butyraldehyd, % | 12,2 | 11,4 |
| Propan, % | 2,0 | 2,0 |
| n/iso-Verhältnis der Aldehyde | 86:14 | 86:14 |

Beispiele 2 und 3

In einem Reaktor von 22 m Länge und einem Durchmesser von 2,80 m wurde Propylen zusammen mit Oxogas zu Butyraldehyden (BA) umgesetzt. Der Versuchsaufbau ist schematisch in der Abbildung dargestellt. Die Einsatzstoffe Propylen und Oxogas wurden zusammen mit einem Teil des Kreisgases bzw. mit der gesamten Kreisgasmenge (Vergleichsbeispiele 2b und 3b) am Boden des Reaktors (1) über eine Verteilungseinrichtung zu der Flüssigphase aus vorwiegend Butyraldehyd-Kondensationsprodukten, die etwa 2/3 des Reaktorvolumens ausfüllte, zugegeben. Ein zweiter Kreisgasstrom wurde über eine analoge Verteilung unterhalb der Flüssigkeitsoberfläche im oberen Viertel der flüssigen Reaktionszone zugeführt.

Der Produktaustrag erfolgte anteilmäßig zum Dampfdruck über das Kreisgas. Das Produkt wurde in einem Kühler (2) auskondensiert und gesammelt (3). Die dabei mit einkondensierten $C_3$-Anteile (Propylen und Propan) wurden in einer separaten Kolonne (4) ausgegast und in dem Kreisstrom zurückgeführt. Die Ausschleusung des Propans erfolgte über einen separaten Abgasstrom (5).

Der nachfolgenden Tabelle 2 sind die erhaltenen Propylenumsätze in Abhängigkeit von den Reaktionsbedingungen wie Art der Zudosierung des Oxogases und Rhodiumkonzentration zu entnehmen. Alle Umsetzungen wurden bei einer Temperatur von 100 °C bei Partialdrucken von $P_{C3H6}$ = 4,8 bar, $P_{H2}$ = 8,0 bar und $P_{CO}$ = 0,6 bar durchgeführt. Das Verhältnis von Rhodium zu Triphenylphosphin betrug jeweils 1 zu 120.

Tabelle 2: Hydroformylierung von Propylen

| Beispiel | Kreisgas kg/h | Zudosierung*) | Rh-Konzentration ppm | Propylen-verbrauch kg/h | Butyraldehydbildung kg/h | n/iso | Propylen-umsatz % |
|---|---|---|---|---|---|---|---|
| 2a | 9270 | unten | 160 | 3997 | 5687 | 84/16 | 83,8 |
| | 13300 | oben | | | | | |
| 2b | 17700 | unten | 163 | 3280 | 4680 | 85/15 | 83,2 |
| 3a | 9390 | unten | 188 | 4399 | 6504 | 86/14 | 86,3 |
| | 16870 | oben | | | | | |
| 3b | 18100 | unten | 190 | 3200 | 4600 | 85/15 | 83,8 |

*) unten = am Boden Reaktors
oben = unterhalb der Flüssigkeitsoberfläche, im oberen Viertel der flüssigen Reaktionszone

## Patentansprüche

1. Verfahren zur kontinuierlichen Hydroformylierung von olefinisch ungesättigten Verbindungen, unter Einleitung des Olefins im unteren Bereich des Reaktors, bei 1 bis 40 bar und 50 bis 140 °C mit Hilfe

von Rhodium-Komplexverbindungen als Katalysatoren in einem Hydroformylierungsreaktor mit einer flüssigen Reaktionszone, die etwa 60 bis 85 % des Reaktorvolumens beträgt, Entfernung der gasförmigen Verfahrensprodukte und Reaktionspartner aus dem Hydroformylierungsreaktor, Abtrennung der Verfahrensprodukte und größtenteils Rückführung des verbleibenden Gases in den Reaktor nach der Kreisgasfahrweise, dadurch gekennzeichnet, daß man 20 bis 80 Vol.% des Kreisgases dem Hydroformylierungsreaktor oberhalb der flüssigen Reaktionszone und/oder unterhalb der Flüssigkeitsoberfläche im oberen Viertel der flüssigen Reaktionszone zuführt.

2. Verfahren nach Anspruch 1, dadurch gekennzeichnet, daß man 40 bis 60 vol. % des Kreisgases unterhalb der Flüssigkeitsoberfläche zuführt.

3. Verfahren nach den Ansprüchen 1 und 2, dadurch gekennzeichnet, daß die Rhodiumkonzentration 120 bis 500 ppm, bezogen auf das flüssige Reaktionsgemisch beträgt.

4. Verfahren nach den Ansprüchen 1 bis 3, dadurch gekennzeichnet, daß 3. man das für die Hydroformylierung benötigte $CO/H_2$-Gemisch aufteilt und die Teilmengen in Ebenen unterschiedlicher Höhe der flüssigen Reaktionszone zuführt.

5. Verfahren nach den Ansprüchen 1 bis 4, dadurch gekennzeichnet, daß man ca. 20 bis 80 Vol.% des $CO/H_2$-Gemisches am Boden des Reaktors zuführt und den Rest auf mindestens einer Ebene oberhalb des unteren Fünftels der flüssigen Reaktionszone.

6. Verfahren nach den Ansprüchen 1 und 5, dadurch gekennzeichnet, daß man es auf die Hydroformylierung von $\alpha$-olefinisch ungesättigten Verbindungen anwendet.

7. Verfahren nach den Ansprüchen 1 bis 6, dadurch gekennzeichnet, daß man $C_1$-$C_4$-Olefine umsetzt.

8. Verfahren nach den Ansprüchen 1 bis 7, dadurch gekennzeichnet, daß die Rhodium-Komplex-Verbindungen schwer flüchtige Verbindungen der Formel I

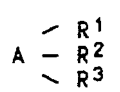 I

in der A für Phosphor, Arsen, Antimon oder Wismut steht und in der $R^1$-$R^3$ organische Reste bedeuten, als Liganden enthalten.

9. Verfahren nach den Ansprüchen 1 bis 7, dadurch gekennzeichnet, daß die Rhodium-Komplexverbindungen Triaryl- oder Aryl-alkylphosphine als Liganden enthalten.

## Claims

1. A process for the continuous hydroformylation of olefinically unsaturated compounds, the olefin being passed into the lower region of the reactor, under from 1 to 40 bar and at from 50 to 140 °C with the aid of a rhodium complex as a catalyst in a hydroformylation reactor having a liquid reaction zone which occupies about 60-85% of the reactor volume, the gaseous products and reactants being removed from the hydroformylation reactor, the products being isolated and the major part of the remaining gas being recycled to the reactor by the cycle gas method, wherein from 20 to 80% by volume of the cycle gas is fed to the hydroformylation reactor above the liquid reaction zone and/or under the liquid surface in the upper fourth of the liquid reaction zone.

2. A process as claimed in claim 1, wherein from 40 to 60% by volume of the cycle gas is fed in below the liquid surface.

3. A process as claimed in claims 1 and 2, wherein the rhodium concentration is from 120 to 500 ppm, based on the liquid reaction mixture.

4. A process as claimed in claim 1 or 2 or 3, wherein the $CO/H_2$ mixture required for the hydroformylation is divided up and the part streams are fed into the liquid reaction zone at different heights.

5. A process as claimed in claim 1 or 2 or 3 or 4, wherein about 20-80% by volume of the $CO/H_2$ mixture is fed in at the bottom of the reactor and the remainder at one or more levels above the bottom fifth of the liquid reaction zone.

6. A process as claimed in claims 1 and 5, which is applied to the hydroformylation of an $\alpha$-olefinically unsaturated compound.

7. A process as claimed in claim 1 or 2 or 3 or 4 or 5 or 6, wherein a $C_1$-$C_4$-olefin is converted.

8. A process as claimed in claim 1 or 2 or 3 or 4 or 5 or 6 or 7, wherein the rhodium complex contains, as a ligand, a sparingly volatile compound of the formula I

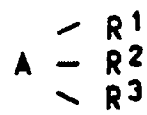

I

where A is phosphorus, arsenic, antimony or bismuth and $R^1$, $R^2$ and $R^3$ are each organic radicals.

9. A process as claimed in claim 1 or 2 or 3 or 4 or 5 or 6 or 7, wherein the rhodium complex contains a triaryl- or aryl-alkylphosphine as a ligand.

**Revendications**

1. Procédé d'hydroformylation en continu de composés à insaturation oléfinique, avec introduction de l'oléfine dans la région inférieure du réacteur, mise en réaction sous une pression de 1 a 40 bar et à une température de 50 à 140° C à l'aide de composés complexes du rhodium servant de catalyseur, dans un réacteur d'hydroformylation comportant une zone de réaction liquide qui constitue 60 à 85% environ du volume du réacteur, évacuation des produits réactionnels et des partenaires réactionnels gazeux hors du réacteur d'hydroformylation, séparation des produits réactionnels et retour de la plus grande partie du gaz résiduaire dans le réacteur suivant le mode opératoire du gaz recyclé, caractérisé en ce qu'on envoie 20 à 80% en volume du gaz recyclé dans le réacteur d'hydroformylation, au-dessus de la zone de réaction liquide et/ou au-dessous de la surface du liquide dans le quart supérieur de la zone de réaction liquide.

2. Procédé selon la revendication 1, caractérisé en ce qu' on envoie 40 à 60% en volume du gaz recyclé audessous de la surface du liquide.

3. Procédé selon la revendication 1 ou 2, caractérisé en ce que la concentration de rhodium est comprise entre 120 et 500 ppm par rapport au mélange réactionnel liquide.

4. Procédé selon l'une quelconque des revendications 1 à 3, caractérisé en ce qu'on partage le mélange $CO/H_2$ nécessaire pour l'hydroformylation et on envoie les quantités partielles dans des plans de hauteurs différentes de la zone de réaction liquide.

5. Procédé selon l'une quelconque des revendications 1 à 4, caractérisé en ce qu'on envoie environ 20 à 80% en volume du mélange $CO/H_2$ au fond du réacteur et on envoie le reste dans un plan au moins situé au-dessus du cinquième inférieur de la zone de réaction liquide.

6. Procédé selon l'une quelconque des revendications 1 a 5, caractérisé en ce qu'on l'applique à l'hydroformylation de composés à insaturation $\alpha$-oléfinique.

7. Procédé selon l'une quelconque des revendications 1 à 6, caractérisé en ce qu'on fait réagir des oléfines en $C_1$-$C_4$.

8. Procédé selon l'une quelconque des revendications 1 à 7, caractérisé en ce que les composés complexes à base de rhodium contiennent, en tant que coordinats, des composés faiblement volatils de formule I

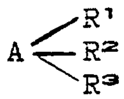

I

dans laquelle A est mis pour un atome de phosphore, d'arsenic, d'antimoine ou de bismuth et dans laquelle $R^1$ a $R^3$ représentent des restes organiques.

9. Procédé selon l'une quelconque des revendications 1 à 7, caractérisé en ce que les composés complexes à base de rhodium contiennent des triaryl- ou arylalkylphosphines en tant que coordinats.

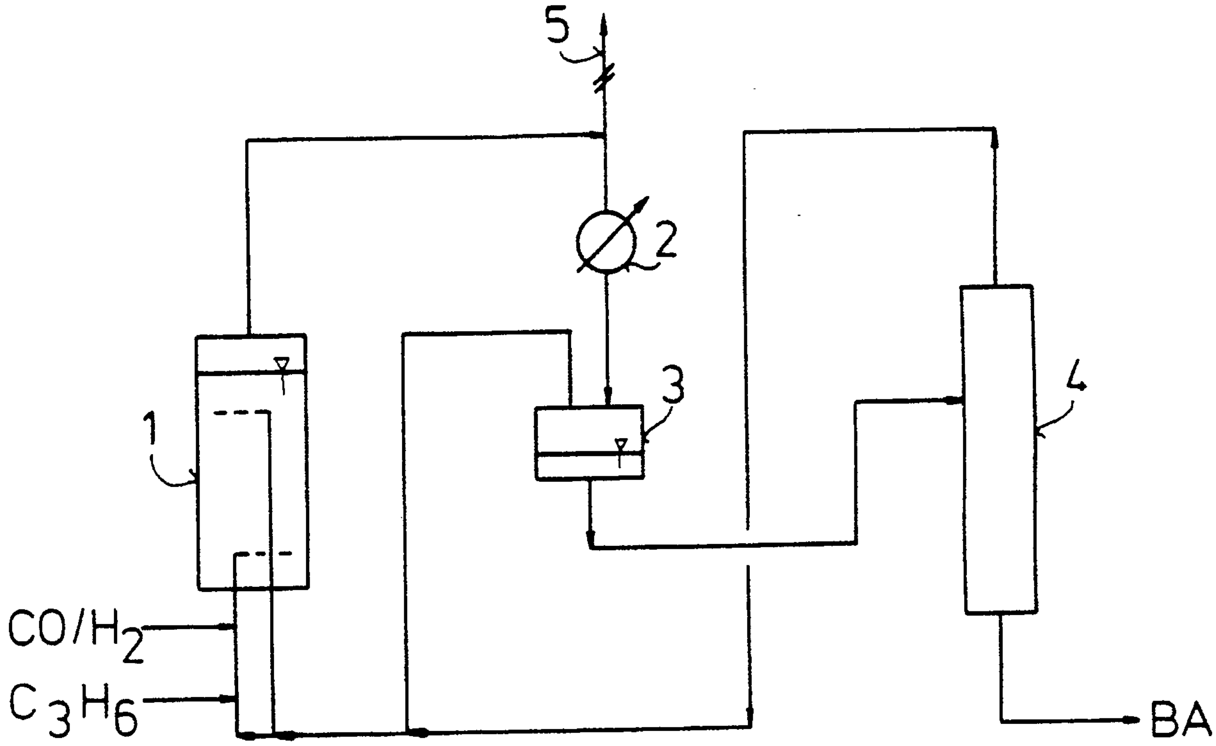
5
2
3
1
4
CO/H2
C3H6
BA